# EUROPEAN PATENT APPLICATION

(11) **EP 2 128 120 A1**
(43) Date of publication of application: **02.12.2009**
(21) Application number: 08009916.1
(22) Date of filing: 30.05.2008
(51) Int. Cl.: C07C 29/56, C07C 33/048

(54) **Process for the rearrangement of allyl alcohols**

(71) Applicant: DSM IP Assets B.V., 6411 TE Heerlen (NL)
(72) Inventor: Aquino, Fabrice, 68950 Reiningue (FR); Bonrath, Werner, 79115 Freiburg (DE)
(74) Representative: Steck, Melanie

(57) **Abstract**

The present invention is directed to a process for isomerizing a pent-1-en-3-ol according to formula (1) 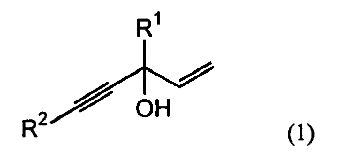 to a mixture of the stereoisomers Z-pent-2-en-1-ol (2) and E-pent-2-en-1-ol (3) according to formulae (2) and (3) 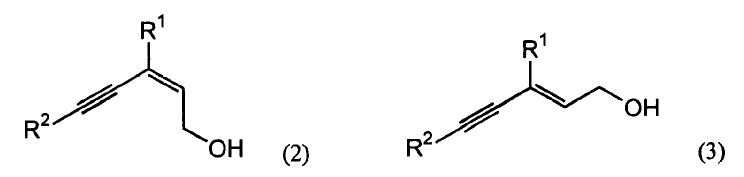 wherein
R¹ is selected from alkyl, aryl, and alkylaryl radicals,
R² is selected from H, alkyl, and aryl radicals;
which process comprises reacting the pent-1-en-3-ol (1) in a solvent and in the presence of a heterogeneous acid catalyst. The heterogeneous acid catalyst is preferably selected from the group consisting of Brønsted acids on a carrier, strong acidic cation exchangers and polymers having acidic groups.

The products of the process according to the present invention are important intermediates for the manufacture of isoprenoids such as vitamin A and derivatives thereof and carotenoids.

## Description

The present invention relates to a process for isomerizing allyl alcohols, especially the rearrangement of a pent-1-en-3-ol to a mixture of the isomers Z-pent-2-en-1-ol and E-pent-2-en-1-ol.

Allyl alcohols, especially tertiary allyl alcohols, are important intermediates in industrial organic chemistry. It is known that allyl alcohols isomerize in presence of an acid catalysis. This isomerization reaction corresponds to a 1,3 migration of the hydroxyl group and a corresponding shift of the double bond. The migration of a double bond and of a substituent is known for allyl compounds and is generally referred to as an allylic rearrangement. Allylic rearrangements of allyl alcohols are equilibrium reactions.

From industrial viewpoint 3-methylpent-1-en-4-yn-3-ol is important and great interest because its allylic rearrangement results in about 85 % of Z-3-methylpent-2-en-4-yn-1-ol and about 15 % of E-3-methylpent-2-en-4-yn-1-ol. The Z-isomer is a useful intermediate, e.g. for the manufacture of Vitamin A, and the E-isomer is also a useful intermediate, e.g. for the manufacture of astaxanthin, zeaxanthin and further carotenoids. The isomers may be separated from each other by physical means, e.g. by fractional distillation.

EP-A-0 860 415 generally describes the isomerization of allyl alcohols to the Z- and E-isomers. The isomerization reaction is performed in an aqueous solution in the presence of protonic acids at a pH in the range of from 2 to 5. The process is predominantly used to convert primary or secondary allyl alcohols to tertiary allyl alcohols. It is further described that a solvent may be present, e.g. in case an allyl alcohol of low solubility in water is used. However, the solvent must be miscible with water in the mixing ratio employed. This method does not seem appropriate to isomerize 3-methylpent-1-en-4-yn-3-ol as this is a tertiary allyl alcohol.

Czech patent application publication no. 269 025 is concerned with a method for producing Z-3-methylpent-2-en-4-yn-1-ol by means of the acid catalyzed allylic rearrangement of 3-methylpent-1-en-4-yn-3-ol. The isomerization reaction is conducted in an organic solvent in the presence of a strongly acidic cation exchanger swollen by water. The organic solvent employed is an aromatic hydrocarbon, e.g. benzene, or a halogenated aliphatic hydrocarbon. However, the reaction is performed at incomplete conversion of no more than 70 % and the maximum yield of 3-methylpent-2-en-4-yn-1-ol reported in the examples is only about 52 %.

In Chem. Papers 43 (6) 743-751 (1989) A. Skoda et al. report on a kinetic study of the allyl rearrangement of 3-methylpent-1-en-4-yn-3-ol catalyzed by sodium hydrogensulfate in aqueous solution. This publication only describes scientific experiments and does not give any hint on a possible realization of a corresponding technical process for large scale production. Moreover, the separation of the catalyst for further reuse is difficult.

It is the object of the present invention to provide an alternative process for isomerizing an allyl alcohol in particular a pent-1-en-3-ol, especially 3-methylpent-1-en-4-yn-3-ol, to a mixture of Z-pent-2-en-1-ol and E-pent-2-en-1-ol, which process provides the isomers in good yields at high conversion and/or with high selectivity. An additional object of the present invention is to establish a catalyst which allows the reducing of waste formation, especially in continuous processing.

The object is met by a process for isomerizing a pent-1-en-3-ol according to formula (1) 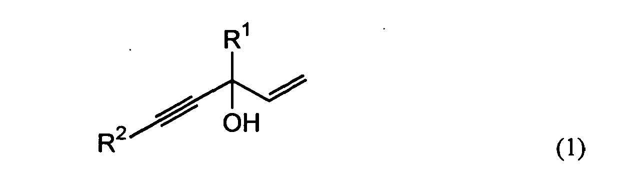 to a mixture of the isomers Z-pent-2-en-1-ol (2) and E-pent-2-en-1-ol (3) according to formulae (2) and (3) 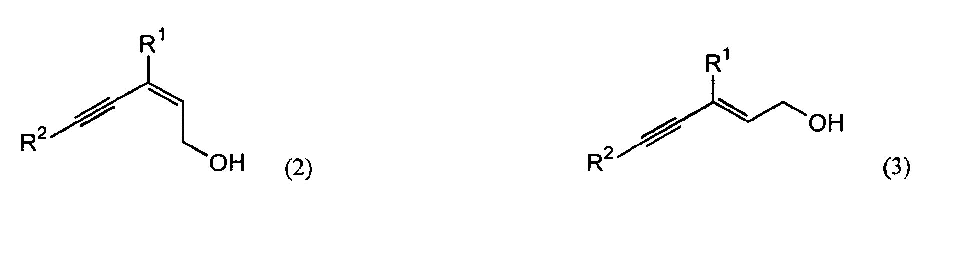 wherein
R¹ is selected from alkyl, aryl, and alkylaryl radicals,
R² is selected from H, alkyl, and aryl radicals;
which process comprises reacting the pent-1-en-3-ol (1) in a solvent and in the presence of a heterogeneous acid catalyst, i.e. that the catalyst is an immobilized solid phase catalyst.

The solvent may either be water or a multiphase system, wherein said multiphase system comprises an aqueous phase and an organic solvent phase which form 2 phases. Such a two-phase liquid solvent system is preferred. In the latter case the reaction mixture is a three-phase system: aqueous phase, organic solvent phase and catalyst.

In an especially advantageous embodiment of the present invention the water content in the reaction mixture is at least 5 weight-%.

In a preferred embodiment the organic solvent phase of said multiphase system comprises (a) water-immiscible organic solvent(s).

The present invention is also directed to a process of preparing an isoprenoid selected from vitamin A, vitamin A derivatives, and carotenoids, which process comprises isomerizing a pent-1-en-3-ol as described above, isolating the Z-pent-2-en-1-ol and E-pent-2-en-1-ol, and converting the Z-pent-2-en-1-ol or E-pent-2-en-1-ol to the corresponding isoprenoid.

The present invention further relates to a process of preparing an isoprenoid selected from vitamin A, vitamin A derivatives, and carotenoids, which process comprises isomerizing 3-methylpent-1-en-4-yn-3-ol as described above, isolating the Z-3-methylpent-2-en-4-yn-1-ol and E-3-methylpent-2-en-4-yn-1-ol, and converting the Z-3-methylpent-2-en-4-yn-1-ol or E-3-methylpent-2-en-4-yn-1-ol to the corresponding isoprenoid.

According to a preferred embodiment, R¹ in formulae (1), (2) and (3) is selected from C₁ to C₁₅ alkyl radicals, preferably C₁ to C₅ alkyl radicals (more preferably methyl radicals), and a phenyl radical; R² is selected from H, C₁ to C₁₅ alkyl radicals, preferably C₁ to C₅ alkyl radicals, and a phenyl radical. The alkyl radicals may be linear, branched or cyclic, preferably they are linear or branched, more preferably they are linear.

Further examples of aryl radicals are naphthyl and tolyl.

An example of an alkylaryl radical is benzyl.

According to the most preferred embodiment, R¹ is a methyl radical and R² is H. In this case, the pent-1-en-3-ol is 3-methylpent-1-en-4-yn-3-ol according to formula (4) 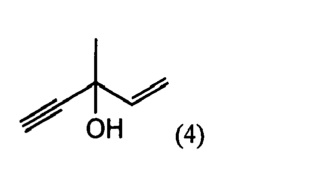 and the corresponding isomers are Z-3-methylpent-2-en-4-yn-1-ol according to formula (5) and E-3-methylpent-2-en-4-yn-1-ol according to formula (6)

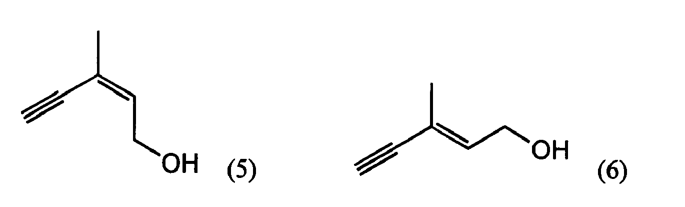

The organic solvent forming the organic solvent phase may be any water-immiscible organic solvent. Mixtures of various water-immiscible organic solvents may also be employed. The term "water-immiscible" solvent means any solvent that maintains during the isomerization reaction a distinct organic solvent phase in addition to the aqueous phase. Typically, the water-immiscible solvent is miscible in water to an extent of less than 3 g per 100 g of water.

Preferably, the water-immiscible organic solvent is a non-halogenated organic solvent. More preferably, the water-immiscible organic solvent is toluene or a non-aromatic organic solvent. In one embodiment the non-aromatic organic solvent may be selected from ethers, ketones, saturated aliphatic hydrocarbons and mixtures thereof. Exemplary organic solvents include diisopropylether, diethyl ether, tetrahydrofuran, methyl isobutyl ketone, hexane, heptane, and paraffin oil. Preferably, the non-aromatic organic solvent is selected from diisopropylether, methyl isobutyl ketone, and paraffin oil. More preferably, the organic solvent is toluene, diisopropylether, methyl isobutyl ketone, and paraffin oil, even more preferably it is diisopropylether or toluene. Most preferably, the organic solvent is diisopropylether.

Preferably, the heterogeneous acid catalyst is selected from the group consisting of Bronsted acids on a carrier, strong acidic cation exchanger and polymers having acidic groups, i.e. functionalized polymers. Preferably the catalyst has a pore diameter of 100 to 500 Ǻ and a surface are of 20 to 500 m²/g (preferably 25 to 300 m²/g, more preferably 30 to 300 m²/g).

Even more preferably the acidic group of the catalyst is represented by sulphonic acid groups (-SO₃H) or by any other group having a pKₐ ≤ 4, preferably the acidic function of the catalyst is represented by sulphonic acid groups.

Thus, the catalyst may be an organic sulphonic acid on a carrier or a polymeric resin with functional sulphonic acid groups, whereby the polymeric resins with functional sulphonic acid groups are more preferred. The polymeric resins with functional sulphonic acid groups are also named "polymer bounded sulfonic acid" in the context of the present invention. In a preferred embodiment a styrene-divinylbenzene polymer is used as matrix for the sulphonic acid groups.

A further suitable catalyst is a polymer functionalized with CO₂H groups.

In the context of the present invention the term "functionalized polymer with SO₃H groups" also encompasses polymers, where the SO₃H groups are bound to the polymer backbone via a linker. This linker may be an alkylene (such as e.g. propylene: -(CH₂)₃-), an arylene (such as p-tolylene) or an alkylarylene (such as e.g. benzylene: -CH₂-(C₆H₄)-). The same applies accordingly for the "functionalized polymer with CO₂H groups".

Of all the functionalized polymers cited in the context of the present invention the macroreticular polymers are preferred.

Preferably, the immobilized/solid Brønsted acid for use in the present invention has a pka value ≤ 4, more preferably ≤ 2, even more preferably ≤ 1.5 and most preferably ≤ 1.

Examples of Brønsted acids are organic sulphonic acids, H₂SO₄ and H₃PO₄.

The carrier may be either organic or inorganic. Examples of organic carriers are polymers. Examples of inorganic carriers are oxide carriers such as e.g. SiO₂, Al₂O₃, TiO₂, ZrO₂ and GeO₂.

Examples of Brønsted acids on a carrier are p-toluene sulphonic acid on a polymer (especially on a macroreticular polystyrene copolymer) or propyl sulphonic acid on silica.

Examples of suitable catalysts for the process of the present invention are Amberlyst type polymers, Levatit, Dowex, und polymer bounded p-TsOH (p-toluene sulphonic acid, as well as Deloxan.

According to the most preferred embodiment, polymer-bounded sulphonic acids, e.g. Amberlyst-type (preferred) and Dowex-type polymers are used as the solid acid catalyst in the present process.

In the most preferred embodiment of the present invention the catalysts Amberlyst 15, 16 and 36, as well as Dowex DR-2030 are used. These are all functionalized styrene divinylbenzene copolymers.

Amberlyst 15 is a bead form, macroreticular, sulphonic acid, strongly acidic ion exchange resin having a concentration of the acid sites (i.e. the sulphonic acid groups) ≥ 4.7 eq/kg, a surface area of 53 m²/g (measured by nitrogen BET), an average pore diameter of 300 Å and a total pore volume of 0.40 ml/g, and commercially available from Rohm & Haas as Amberlyst 15 Dry and Amberlyst 15 Wet. Amberlyst 15 Wet has also a harmonic mean particle size in the range as from 0.600 to 0.850 mm and a uniformity coefficient ≤ 1.70.

Amberlyst 16 is a bead form, macroreticular, sulphonic acid, strongly acidic ion exchange resin having a concentration of the acid sites (i.e. the sulphonic acid groups) ≥ 4.8 eq/kg, a surface area of 30 m²/g (measured by nitrogen BET), an average pore diameter of 250 Å and a total pore volume of 0.20 ml/g, and commercially available from Rohm & Haas as Amberlyst 16 Wet. Amberlyst 16 Wet has also a harmonic mean particle size in the range as from 0.600 to 0.800 mm and a uniformity coefficient ≤ 1.60.

Amberlyst 36 is a bead form, macroreticular, sulphonic acid, strongly acidic ion exchange resin having a concentration of the acid sites (i.e. the sulphonic acid groups) ≥ 5.4 eq/kg, a surface area of 33 m²/g (measured by nitrogen BET), an average pore diameter of 240 A and a total pore volume of 0.20 ml/g, and commercially available from Rohm & Haas as Amberlyst 36 Dry and Amberlyst 36 Wet. Amberlyst 36 Wet has also a harmonic mean particle size in the range as from 0.600 to 0.850 mm and a uniformity coefficient ≤ 1.60.

Dowex DR-2030 is a bead form, macroreticular, sulphonic acid, strongly acidic ion exchange resin having a concentration of the acid sites (i.e. the sulphonic acid groups) ≥ 4.7 eq/kg, a surface area of 30-31 m²/g (measured by nitrogen BET) and a total pore volume of 0.33-0.35 ml/g, and commercially available from Dow Chemical as Dowex DR-2030 and Dowex Monosphere DR-2030. Dowex Monosphere DR-2030 has also a particle size distribution of 425-525 microns.

Thus, the most preferred heterogeneous acid catalysts of the present invention are bead form, macroreticular, sulphonic acid, strongly acidic ion exchange resin having a concentration of the acid sites (i.e. the sulphonic acid groups) ≥ 4.0 eq/kg, a surface area of at least 20 m²/g (measured by nitrogen BET) (preferably in the range of from 20 to 70 m²/g), an average pore diameter of at least 200 Å (preferably in the range of from 200 to 350 A)and a total pore volume of at least 0.10 ml/g (preferably in the range of from 0.10 to 0.50 ml/g).

Furthermore polymers with SO₃H groups as prepared according to the processes of EP-A 507 490 and US 5,081,160, whose contents are incorporated herein by reference, may be also used as catalyst in the isomerization reaction according to the present invention.

Although it is not mandatory, a stabilizer, such as hydroquinone or 2,6-di-tert-butyl-p-cresol or a mixture thereof, may be added to the reaction mixture. If used, the stabilizer, e.g. hydroquinone or 2,6-di-tert-butyl-p-cresol or a mixture thereof, is typically added in an amount of from 1 to 10,000 ppm by weight, preferably from 5 to 1,000 ppm by weight, more preferably from 7 to 500 ppm by weight, and most preferably from 10 to 100 ppm by weight, each based on the total weight of the multiphase system.

Further stabilizers may be other anti-oxidants.

Typically, the solid acid catalyst is mixed with water and added to the process.

Compared to the prior art methods the separation and recycling of the catalyst is much more convenient in the present process. If only water is used as solvent for the reaction, the water and the catalyst may be separated from the product, which may be distilled for further purification. If a multiphase system is used, the solid catalyst and the aqueous phase of the multiphase system may easily be separated from the product layer and recycled. That means that in both variants the catalyst may be recycled. It has been found that the catalyst gives still high yield and conversion even if used several times.

Usually, the pent-1-en-3-ol is soluble in the water-immiscible non-aromatic non-halogenated organic solvent and thus, it is added to the process as a solution in the organic solvent. In one embodiment, the concentration of the pent-1-en-3-ol in the organic solvent ranges from 5 to 80 % by weight, preferably from 15 to 60 % by weight, more preferably from 20 to 50 % by weight, and most preferably from 25 to 45 % by weight.

The pent-1-en-3-ol may, however, be also added in the reaction chamber as such. Preferably the pent-1-en-3-ol is added as a solution in a water-immiscible non-aromatic non-halogenated organic solvent.

The various components may be added in any order. For example, the aqueous mixture of the solid acid catalyst may be loaded into the reaction vessel and then the solution of the pent-1-en-3-ol in the organic solvent may be added or vice versa. It is also possible to divide the total amount of a component to be added and add the partial amounts at various stages in the process.

In another embodiment of the present invention, where not a multiphase system is used but only water, the pent-1-en-3-ol is used as such and may be added to the mixture of catalyst and water or vice versa. Here it is also possible to divide the total amount of a component to be added and add the partial amounts at various stages in the process.

If the reaction is carried out discontinuously the mixing of the reaction components is done by agitation of the reaction mixture, e.g. by stirring. Preferably, the isomerization reaction is conducted in a stirred tank reactor.

If the reaction is carried out in continuous manner a plough-flow reactor with a fixed-bed is used, or a continuous stirred tank reactor. In an embodiment of the invention a cascade reactor system is used.

The isomerization reaction is typically conducted at a temperature in the range of from 20 to 80°C, depending on the type of the reaction components used and the other reaction conditions applied. At atmospheric pressure the reaction temperature preferably ranges from 30 to 70°C, more preferably from 35 to 65°C, and most preferably from 40 to 60°C.

The isomerization reaction has depending of the used set-up and conditions reaction times from 1 to 25 h. At a typical temperature range the reaction time is preferably from 1.5 to 8 h, more preferably from 2 to 6 h, and most preferably from 2 to 5 h.

Typically, the process according of the present invention is conducted at a pent-1-en-3-ol conversion of at least 70 %, preferably at least 81 %, more preferably at least 86 %, even more preferably at least 89 % and most preferably at least 92 %.

In a preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol using the reaction components, amounts and reaction conditions described above, including all the preferred embodiments.

In a more preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as its/their solvent and using the other reaction components, amounts and reaction conditions described above, including all the preferred embodiments.

In another preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as is/their solvent, Brønsted acids on a carrier, strong acidic cation exchanger or polymers having acidic groups as the solid acid catalyst, whereby the catalyst has a pore diameter of 100 to 500 Ǻ and a surface are of 20 to 500 m²/g (preferably 25 to 300 m²/g, more preferably 30 to 300 m²/g), and using the other reaction components, amounts and reaction conditions described above, including all the preferred embodiments.

In another more preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing polymer bounded sulfonic acid as the solid acid catalyst and using the other reaction components, amounts and reaction conditions described above, including all the preferred embodiments.

In an even more preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as is/their solvent, polymer bounded sulfonic acid as the solid acid catalyst and using the other reaction components, amounts and reaction conditions described above, including all the preferred embodiments.

In a further preferred embodiment of the present invention 3-methylpent-1-en-4-yn-3-ol is isomerized to a mixture of Z-3-methylpent-2-en-4-yn-1-ol and E-3-methylpent-2-en-4-yn-1-ol employing diisopropylether as is/their solvent, Amberlyst or Dowex type polymers as the solid acid catalyst and using the other reaction components, amounts and reaction conditions described above, including all the preferred embodiments.

If the process of the present invention is carried out in a multiphase system, the reaction mixture obtained from the isomerization step comprises an aqueous phase and an organic solvent phase. In the further work-up a phase separation step followed by an optionally neutralization, e.g. with sodium carbonate, and separation of the organic solvent and not reacted tertiary allyl alcohol, e.g. pent-1-en-3-ol, is known in the art. Typically, the lower boiling components (e.g. organic solvent) and higher boiling components will be separated from the isomer mixture in one or more, preferably at least two devolatilization and/or distillation steps. Typically, the Z-pent-2-en-1-ol is then separated from the E-pent-2-en-1-ol by one or more final rectification steps.

The present invention also relates to a process of preparing an isoprenoid selected from vitamin A, vitamin A derivatives, and carotenoids, which process comprises isomerizing a pent-1-en-3-ol as described above, isolating the Z-pent-2-en-1-ol and E-pent-2-en-1-ol, and converting the Z-pent-2-en-1-ol or E-pent-2-en-1-ol to the corresponding isoprenoid.

Z-3-methylpent-2-en-4-yn-1-ol obtained in major amounts by a preferred embodiment of the present isomerization reaction is an intermediate product for the syntheses of vitamin A or vitamin A derivatives. One of the most economically successful processes for the preparation of vitamin A and its derivatives is the Isler synthesis of 1948. The conversion of Z-3-methylpent-2-en-4-yn-1-ol to vitamin A or a vitamin A derivative is for example described in US-A-2,451,739 to Otto Isler. E-3-methylpent-2-en-4-yn-1-ol obtained in minor amounts by the preferred embodiment of the present isomerization reaction also is a useful intermediate. It may be used to synthesize astaxanthin, zeaxanthin and further carotenoids. An illustrative synthesis of astaxanthin is described in EP-A-0 005 748. A different synthesis of astaxanthin also using E-3-methylpent-2-en-4-yn-1-ol as starting C₆ component is taught in CN-A-166 803.

The invention will now be further illustrated in the following non-limiting examples.

### EXAMPLES

In the following examples the terms "yield" and "selectivity" both refer to the sum of the desired products, i.e. the sum of the Z- and E-isomers.

The reactions were carried out in a flask equipped with a stirrer (mechanical or magnetical), a thermometer and a reflux condenser with Ar overlay. This will be heated with an oil bath filled with WACKER Silicon oil AP 100.

### Chemicals

Pure 3-methylpent-1-en-4-yn-3-ol (97%), as well as 46, 48 and 50 weight-% solutions of 3-methylpent-1-en-4-yn-3-ol in iPr₂O (diisopropylether) were obtained from DSM Nutritional Products Ltd, Sisseln, Switzeland. Diisopropylether (99%) itself was also obtained therefrom. It was stabilized with 2,6-di-ter-butyl-p-cresol (99%), which was purchased from Fluka.

The catalysts Amberlyst® 15, Amberlyst® 16 and Amberlyst® 36 were purchased from Rohm & Haas, (Springhouse, Philadelphia, USA).

The catalyst Dowex DR-2030 was purchased from Fluka.

### Example 1: Procedure for batch experiments in water/diisopropyl ether

In the reactor, 20 g dry solid acid catalyst (Amberlyst 36 Dry) was placed. Under stirring at 350 rpm at 294 K 100 ml of water was added. A mixture of 100 ml 50 weight-% 3-methylpent-1-en-4-yn-3-ol (0.42 mol) in diiso-propyl ether was added. The reaction mixture was heated up to 328 K and stirred for 5 h. After cooling to room temperature the solid phase was separated from the liquid phases and washed two times with 30 ml diisopropyl ether. The water phase was separated from the organic layer and replaced in the reaction flask with the catalyst. The organic layer was washed two times with 10 ml water and concentrated at 200 mbar, 35°C in 30 min. The crude product (60 g) was analyzed by GC with internal standard. E- and Z-3-methylpent-2-en-4-yn-1-ol could be synthesized at 98 % conversion in 90 % yield.

### Example 1a

Example 1 was repeated with 40 g of Amberlyst 36 Wet and 80 ml water. The result was the same.

### Example 2: Procedure for batch experiments in water

In the reactor, 35.2 g pure 3-methylpent-1-en-4-yn-3-ol (0.36 mol) was stirred (350 rpm) at room temperature in presence of 100 ml water and 20 g catalyst (Amberlyst 15 Dry). The reaction mixture was heated up to 323 K and stirred for 7 h. After cooling to 294 K the solid phase was separated from the liquid phases and washed two times with 50 ml diisopropyl ether. The water phase was separated from the organic layer and replaced in the reaction flask with the catalyst. The organic layer was washed with 3 ml saturated Na₂CO₃ solution and concentrated at 200 mbar, 308 K (30 min). The crude product (50 g) was analyzed by GC with internal standard. E- and Z-3-methylpent-2-en-4-yn-1-ol could be synthesized at 98 % conversion in 90 % yield.

### Example 2a

Example 2 was repeated with 40 g of Amberlyst 15 Wet and 80 ml water. The result was the same.

### Example 3: General procedure for the continuous process

The plugh-flow reactor was filled with the catalyst (Amberlyst 36) and the 3-methylpent-1-en-4-yn-3-ol in a multiphase solvent, e.g a diisopropyl ether water mixture, was pumped through the static mixer and the reactor. After the reaction mixture has passed the reactor it was collected and analyzed. The reactor jacket was heated to the desired reaction temperature. Samples were taken for GC-analysis to follow the progression of the reaction. The catalyst life-time was > 150 hours without loss on activity and selectivity.

### Example 4: Procedure for batch experiments in water/diisopropyl ether

In the reactor, 20 g dry solid acid catalyst (Dowex DR-2030) was placed. Under stirring at 350 rpm at 294 K 100 ml of water was added. A mixture of 100 ml 50 weight-% 3-methylpent-1-en-4-yn-3-ol (0.42 mol) in diisopropyl ether was added. The reaction mixture was heated up to 328 K and stirred for 5 h. After cooling to room temperature the solid phase was separated from the liquid phases and washed two times with 30 ml diisopropyl ether. The water phase was separated from the organic layer and replaced in the reaction flask with the catalyst. The organic layer was washed two times with 10 ml water and concentrated at 200 mbar, 35°C in 30 min. The crude product (60 g) was analyzed by GC with internal standard. E- and Z-3-methylpent-2-en-4-yn-1-ol could be synthesized at 95 % conversion in 86 % yield.

### Example 5: Recycling of catalyst

In a series of experiments with the experimental set-up as described in examples 1, 2 and 4, the catalyst (Amberlyst 36, Amberlyst 15, Amberlyst 16, Dowex DR-2030) were re-used after separation of the reaction mixture. The catalysts could be re-used in minimum 5 times with out decrease in selectivity and yield.

| **Catalyst** | **conversion (after 5^{th} run) in %** | **yield (after 5^{th} run) in %** |
|---|---|---|
| Amberlyst 36 | 96 | 90 |
| Amberlyst 15 | 98 | 83 |
| Amberlyst 16 | 96 | 86 |
| Dowex DR20-30 | 95 | 84 |

## Claims

1. A process for isomerizing a pent-1-en-3-ol according to formula (1) 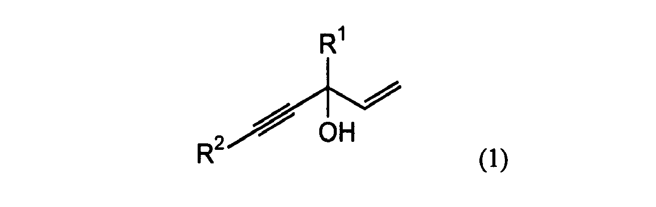 to a mixture of the stereoisomers Z-pent-2-en-1-ol (2) and E-pent-2-en-1-ol (3) according to formulae (2) and (3) 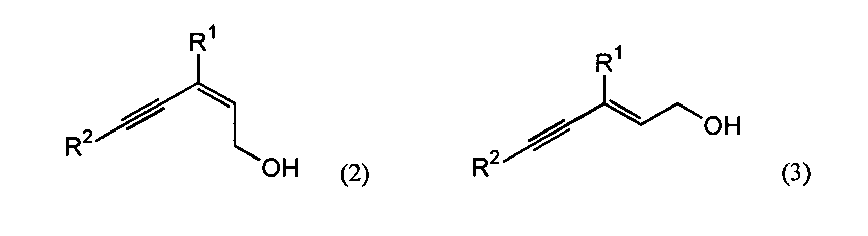 wherein
R¹ is selected from alkyl, aryl, and alkylaryl radicals,
R² is selected from H, alkyl, and aryl radicals;
which process comprises reacting the pent-1-en-3-ol (1) in a solvent and in the presence of a heterogeneous acid catalyst.

2. The process according to claim 1 wherein R¹ is selected from C₁ to C₁₅ alkyl radicals and a phenyl radical, R² is selected from H, C₁ to C₁₅ alkyl radicals and a phenyl radical.

3. The process according to claim 2 wherein R¹ is a methyl radical and R² is H.

4. The process according to any of claims 1 to 3 wherein the solvent is water.

5. The process according to any of claims 1 to 3 wherein the solvent is a multiphase system comprising an aqueous phase and an organic solvent phase.

6. The process according to claim 5 wherein the organic solvent phase comprises a water-immiscible organic solvent.

7. The process according to claim 6 wherein the water-immiscible organic solvent is selected from ethers, ketones, saturated aliphatic hydrocarbons, and mixtures thereof.

8. The process according to any one ore more of the preceding claims wherein the heterogeneous acid catalyst is selected from the group consisting of Brønsted acids on a carrier, strong acidic cation exchangers and polymers having acidic groups.

9. The process according to claim 8 wherein the heterogeneous acid catalyst is a polymer bonded sulfonic acid.

10. The process according to any of claims 1-9 wherein the heterogeneous acid catalyst has a pKa-value ≤ 4, more preferred ≤ 2, especially preferred ≤ 1.5.

11. The process according to any one or more of the preceding claims wherein the catalyst has a pore diameter of 100 to 500 Ǻ and a surface area of 20 to 500 m²/g.

12. The process according to any of the preceding claims wherein the pent-1-en-3-ol is reacted in the presence of a stabilizer, preferably in the presence of hydroquinone and/or 2,6-di-tert-butyl-p-cresol.

13. The process according to any of the preceding claims further comprising isolating the Z-pent-2-en-1-ol and E-pent-2-en-1-ol.

14. A process of preparing an isoprenoid selected from vitamin A, vitamin A derivatives, and carotenoids, which process comprises isomerizing a pent-1-en-3-ol according to any of the preceding claims, isolating the Z-pent-2-en-1-ol and E-pent-2-en-1-ol, and converting the Z-pent-2-en-1-ol or E-pent-2-en-1-ol to the corresponding isoprenoid.

15. The process according to claim 14 wherein the carotenoids are astaxanthin and zeaxanthin.
